# EUROPEAN PATENT SPECIFICATION

(11) **EP 2 043 523 B1**
(45) Date of publication and mention of the grant of the patent: **14.08.2013**
(21) Application number: 06824408.6
(22) Date of filing: 13.12.2006
(51) Int. Cl.: A61B 5/151, A61B 19/02

(54) **LANCET**
LANZETTE
LANCETTE

(30) Priority: 29.03.2006 PL 37932406
(43) Date of publication of application: 08.04.2009
(73) Proprietor: "HTL-Strefa" Spolka Akcyjna, 95-035 Ozorkow (PL)
(72) Inventor: SARNA, Wojciech, PL-02-739 Warszawa (PL); JANKOWSKI, Andrzej, PL-01-390 Warszawa (PL); WYSZOGRODZKI, Wojciech, PL 01-541 Warszawa (PL)
(74) Representative: Vossius & Partner
(86) International application number: PCT/PL2006/000090
(87) International publication number: WO 2007/111519

(56) References cited:
- EP-A- 1 405 596
- EP-A- 1 632 179
- US-A- 4 889 117

## Description

### Technical Field

The subject of the invention is a lancet, in particular for a patient's skin puncturing device for collecting a blood sample for diagnostic purposes.

### Background Art

The patent application EP 1632179 A1 discloses a lancet according to the preamble of claim 1. From the patent No. US 3,358,689 (HIGGINS, John L.) is known a lancet comprised of a plastic body, in which a needle is fixed. The needle has a tip extending away from an abutment surface of the body. The needle tip is sealably encapsulated in a unitary sheath, which is formed integrally and sealably with the body and joined therewith by a frangible connection. Twisting or pulling of the sheath and its rotating about a longitudinal axis of the lancet body enables a fracture of the frangible connection and a removal of the sheath from the needle tip. The needle with the tip are protected against a loss of a sterility until the sheath is removed from the needle tip.

From the international publication WO 2003/015603 is known a plastic lancet comprising a body, in which a needle is arranged. The body has at least one hole exposing the crooked needle. The needle tip extends away from the body and is covered by a sheath moulded together with the body during manufacturing process, whereas a connection between the sheath and the body is fragile. Twisting or pulling of the sheath relative the body enables a break of the sheath away the body and uncovering the needle tip for puncturing the patient's skin. The needle tip is provided with the sterility until the sheath is removed from the needle tip.

In the cited known lancets the tip of the needle, in particular of the needle of a small diameter, is not protected against a bend or damage during the removal of the sheath from the needle tip. The lancet with the damaged tip is not suitable to be used. This is because such a damage of the tip prior to use of the lancet for a puncture of the skin precludes from performing a blood sample drawing correctly and possibly with little painful for the patient The more, an incidental use of such a damaged lancet could be unpleasant and even dangerous for the patient.

The patent US 4,577,630 (NITZSCHE, Raymond P. et al.) teaches a lancet for applying in a lancet firing device. The lancet comprises a body with an extending outwardly needle and a removable sheath, which protects the cleanliness and sharp edges of the needle tip from contamination and damage before use of the lancet. The sheath is removed before the lancet use and the lancet is inserted into sled of the firing device. After the lancet use, the sheath may be reinstalled onto the needle tip to protect the patient and subsequent lancet users from inadvertent skin puncture.

In this known lancet the needle tip is poorly protected against contamination and does not provide the sterility required for medical applications. This is due to the lack of the moulded during the manufacturing process integral connection between the sheath of the needle tip and the body, in which the needle is fixed.

The known lancets do not insure the required sterility and, simultaneously, the correctness of setting of the designed needle tip during puncturing the patient's skin and blood sample drawing. The initial step of the lancet preparation to being used, which consists in the sheath removal from the tip by the user, produces a menace to the patient The known lancets provide the users with a functional discomfort connected with the lack of certainty that the sheath has been correctly removed and the tip is free from damage and sterile.

### Disclosure of Invention

The object of the present invention to provide a cheap lancet of a simple structure, which guarantees the sterility required for the needle tip and, simultaneously, precludes the needle tip from being bent or damaged, particularly the needle of the small diameter, during removal of the sheath from the needle tip.

The essence of the lancet according to the present invention, in particular for a patient's skin puncturing device for collecting a blood sample for diagnostic purposes, comprised of a plastic body, in which a needle is coaxially fixed, whereas a tip of the needle is covered by a sheath moulded together with the body and breakably connected therewith, is that it has additional and coaxial with said body and said sheath a guiding member, which is slidably mounted on a guiding segment of said body and fixed to said sheath.

Preferably, said guiding member constitutes a cap having detention means on an inner surface, and said sheath has on a circumferential outer surface protrusions engaging with said cap and said sheath has a detention edge mating with said detention means of said cap.

Preferably, said detention means constitute a circumferential catch.

Preferably, said cap has on a circumferential outer surface projections to facilitate gripping.

Preferably, said sheath has on a circumferential outer surface additional projections to facilitate gripping.

The guiding member, which during the removal of the sheath and uncovering of the needle tip moves together with the sheath axially lengthwise the body, precludes from deflecting of the sheath and thereby secures the needle tip against bend and damage.

The advantage of the lancet according to the present invention is that its structure insures the required sterility of the needle tip and, simultaneously, the protection, in particular, in case of the needle of small diameter, from damage during the removal of the sheath from the needle tip.

The next advantage of the present lancet is that it ensures for the user and the patient a high functional comfort during use.

Moreover, the simple structure of the present lancet provides a cheap end product, what is highly beneficial in case of devices intended for being used in a wide variety of medical applications, in particular, in view of the need of the mass production of such devices and their availability for a wide circle of professional and individual consumers.

### Brief description of drawings

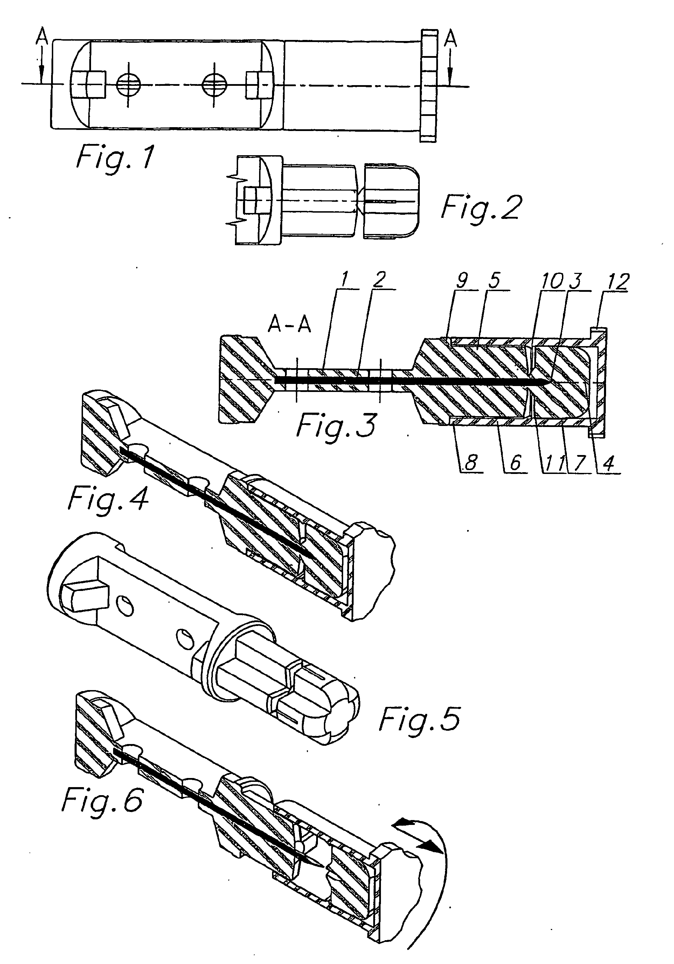

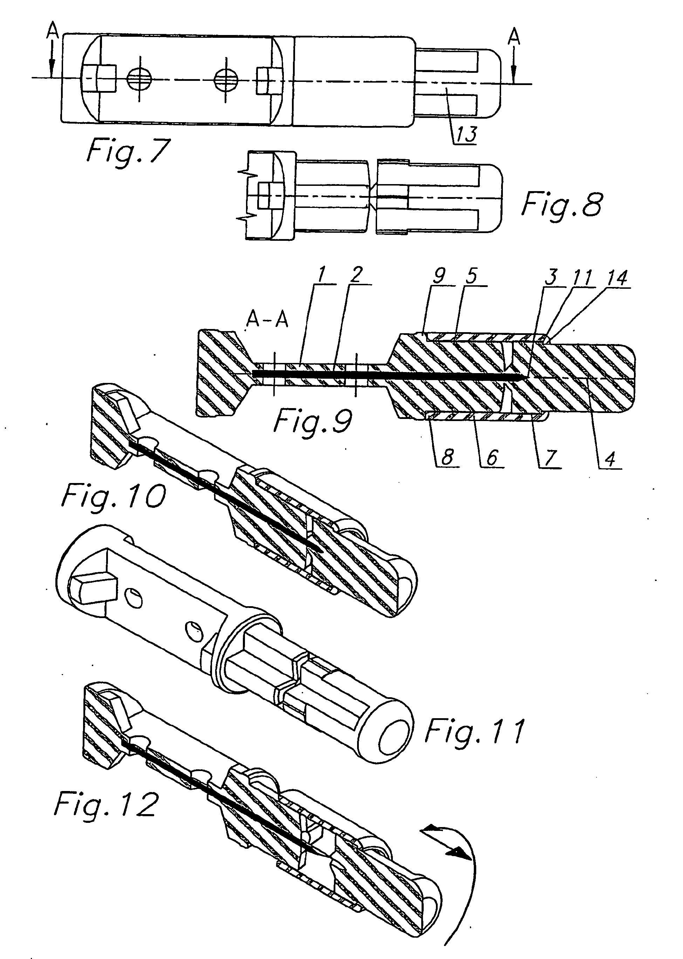

The subject of the invention is presented in examplary embodiments in the drawings, where Fig. 1 and Fig. 7 show the lancet, respectively, in the first and in the second embodiment of its realization, from the top view, Fig. 2 and Fig. 8 show the fragment of the lancet body with the sheath, prior to mounting the guiding member over, respectively, in the first and in the second embodiment of its realization, from the top view, Fig. 3 and Fig. 9 - the sectional view along the line A-A of the lancet in Fig. 1 and Fig. 7, respectively, in the first and in the second embodiment of its realization, Fig. 4 and Fig. 10 - the perspective view of the lancet half cut along the line A-A in Fig. 1 and Fig. 7, respectively, in the first and in the second embodiment of its realization, Fig. 5 and Fig. 11 - the perspective view of the lancet with the sheath, prior to mounting the guiding member over, respectively, in the first and in the second embodiment of its realization, and Fig. 6 and Fig. 12 - the perspective view of the lancet half cut along the line A-A in Fig. 1 and Fig. 7, respectively, in the first and in the second embodiment of its realization, during the removal of the sheath.

### Best Mode of Carrying Out the Invention

In Fig. 1 to Fig. 6 the lancet according to the present invention is shown, in the first preferred embodiment of its realization. The lancet, which before being used is settled in the handle of the patient's skin puncturing device, is comprised of the body 1 made of plastic material, in which is coaxially fixed the needle 2 made of a solid stainless steel. The tip 3 of the needle 2 is covered by the sheath 4 moulded together with the body 1 during manufacturing process. The connection between the sheath 4 and the body 1 is fragile. Onto the sheath 4 and the guiding segment 5 of the body 1 a cap 6 is slid over. During sliding over, the cap 6 is being firmly and coaxially fixed relative the sheath 4 by mutual engaging with the outward protrusions 7 of the sheath 4. After being mounted on the body 1 and the sheath 4, the cap 6 abuts its end surface 8 on its one end against the abutting surface 9 of the body 1, whereas the circumferential catch 10 on an inner surface of the cap 6 catches on the detention edge 11 of the sheath 4. The cap 6 is slidably mounted on the guiding segment 5 of the body 1. In this state, the lancet is subjected to the irradiation process for the sterilization of the tip 3 of the needle 2. For uncovering of the sterile tip 3 of the needle 2, the cap 6 should at first be turned relative the body 1 by gripping by the user's fingers the projections 12 situated on the circumferential outer surface of the cap 6, and then the cap 6 should be pulled away the guiding segment 5 of the body 1, in accordance with arrows, as it is depicted in Fig. 6. Due to the cap 6 being engaged with the outward protrusions 7 of the sheath 4, while turning the cap 6, also the sheath 4 is turned and is broken away the body 1. Due to the slide guidance on the guiding segment 5 of the body 1 the cap 6 moves during its removal from the body 1 along the axis of the body 1 and the needle 2 and by means of the catch 10 takes the sheath 4 away from the tip 3 of the needle 2. In this manner the axial movement of the sheath 4 relative the tip 3 of the needle 2 is guaranteed during the removal of the sheath 4 from the tip 3. When the whole sheath 4 is removed from the tip 3 of the needle 2 the cap 6 is further removed from the guiding segment 5 of the body 1 until its complete separation from the body 1. Next, the lancet for its use is settled in the patient's skin puncturing device.

In Fig. 7 to Fig. 12 the lancet according to the present invention is shown, in the second preferred embodiment of its realization. This lancet, similarly, as in the first preferred embodiment, is comprised of the body 1 made of plastic material, in which is coaxially fixed the needle 2 made of the solid stainless steel. The tip 3 of the needle 2 is covered by the sheath 4 moulded together with the body 1 during manufacturing process. The connection between the sheath 4 and the body 1 is fragile. Onto the sheath 4 and the guiding segment 5 of the body 1 the cap 6 is slid over. During sliding over the cap 6 is being firmly and coaxially fixed relative the sheath 4 by mutual engaging with the outward protrusions 7 of the sheath 4. After being mounted on the body 1 and the sheath 4, the cap 6 abuts its end surface 8 on its one end against the abutting surface 9 of the body 1 and is slidably mounted on the guiding segment 5 of the body 1. In this state, the lancet is subjected to the irradiation process for sterilization of the tip 3 of the needle 2. For uncovering the sterile tip 3 of the needle 2, the sheath 4 should be at first turned relative the body 1 by gripping by the user's fingers the additional projections 13 situated on the circumferential outer surface of the sheath 4 until breaking the sheath 4 away the body 1, and next the sheath 4 should be removed together with the cap 6 from the guiding segment 5 of the body 1, in accordance with arrows, as it is depicted in Fig. 12. The removal of the cap 6 by the sheath 4 is possible due to the circumferential rim provided with the detention edge 11 on the circumferential outer surface of the sheath 4. The detention edge 11 abuts against a circumferential catch 14 on an inner surface of the cap 6 and at its second end. Due to the slide guidance on the guiding segment 5 of the body 1 the cap 6 moves during its removal along the axis of the lancet and ensures the axial movement of the sheath 4 relative the tip 3 of the needle 2 during the removal of the sheath 4 from the tip 3. When the whole sheath 4 is removed from the tip 3 of the needle 2 the cap 6 is further removed from the guiding segment 5 of the body 1 until its complete separation from the body 1. Next, for being used the lancet is settled in the patient's skin puncturing device.

On the basis of the above examplary embodiments of the invention, it is possible to provide its different variants changed, modified and improved, while such changes, modifications and improvements are obvious in the light of the idea of the invention and the accompanying patent claims.

## Claims

1. Lancet, particularly for a patient's skin puncturing device for collecting a blood sample for diagnostic purposes, comprised of
a plastic body (1), in which a needle (2) is coaxially fixed,
whereas a tip (3) of the needle (2) is covered by a sheath (4) moulded together with the body (1) and breakably connected therewith,
a guiding member (6) coaxially with said body (1) and said sheath (4),
said guiding member (6) being slidably mounted on a guiding segment (5) of said body (1) and firmly and coaxially fixed to said sheath (4),
**characterized by** an inner surface of said guiding member (6) and outer surfaces of said guiding segment (5) and said sheath (4) provide a slide guidance of said guiding member (6) during mounting of said guiding member (6) onto said guiding segment (5) and by an inner surface of said guiding member (6) and an outer surface of said guiding segment (5) provide a slide guidance of said guiding member (6) during removal of said guiding member (6) from said guiding segment (5) to guarantee axial movement of said sheath (4) relative to said tip (3) during removal of said sheath (4) from said tip (3).

2. Lancet according to claim 1, **characterized in that** said guiding member constitutes a cap (6) having detention means on an inner surface, and said sheath (4) has on a circumferential outer surface protrusions (7) engaging with said cap (6) and said sheath (4) has a detention edge (11) mating with said detention means of said cap (6).

3. Lancet according to claim 2, **characterised in that** said detention means constitute a circumferential catch (10, 14).

4. Lancet according to claim 2 or 3, **characterised in that** said cap (6) has on a circumferential outer surface projections (12) to facilitate gripping.

5. Lancet according to claim 2 or 3, **characterised in that** said sheath (4) has on a circumferential outer surface additional projections (13) to facilitate gripping.

## Patentansprüche

1. Lanzette, insbesondere für eine Punktionsvorrichtung für die Haut eines Patienten zum Sammeln einer Blutprobe zur Diagnosezwecken mit:
einem Kunststoffkörper (1), in dem koaxial eine Nadel (2) befestigt ist,
wobei eine Spitze (3) der Nadel (2) durch eine Umhüllung (4) abgedeckt ist, die zusammen mit dem Körper (1) ausgeformt und brechbar damit verbunden ist,
einem Führungselement (6), das mit dem Körper (1) und der Umhüllung (4) koaxial ist,
wobei das Führungselement (6) verschiebbar an einem Führungssegment (5) des Körpers (1) angebracht und stabil und
koaxial an der Umhüllung (4) befestigt ist,
**dadurch gekennzeichnet, dass**
eine Innenfläche des Führungselements (6) und Außenflächen des Führungssegments (5) und die Umhüllung (4) während der Befestigung des Führungselements (6) am Führungssegment (5) eine Gleitführung des Führungselements (6) bereitstellen und dass eine Innenfläche des Führungselements (6) und eine Außenfläche des Führungssegments (5) während der Entfernung des Führungselements (6) vom Führungssegment (5) eine Gleitführung des Führungselements (6) bereitstellen, um eine axiale Bewegung der Umhüllung (4) relativ zur Spitze (3) während der Entfernung der Umhüllung (4) von der Spitze (3) sicherzustellen.

2. Lanzette nach Anspruch 1, **dadurch gekennzeichnet, dass** das Führungselement eine Kappe (6) mit einer Arretierungseinrichtung an einer Innenfläche bildet, und die Umhüllung (4) auf einer Umfangsaußenfläche Vorsprünge (7) aufweist, die mit der Kappe (6) in Eingriff treten, und die Umhüllung (4) eine Arretierungskante (11) aufweist, die mit der Arretierungseinrichtung der Kappe (6) zusammenpasst.

3. Lanzette nach Anspruch 2, **dadurch gekennzeichnet, dass** die Arretierungseinrichtung eine Umfangsraste (10, 14) bildet.

4. Lanzette nach Anspruch 2 oder 3, **dadurch gekennzeichnet, dass** die Kappe (6) auf einer Umfangsaußenfläche Vorsprünge (12) aufweist, um das Greifen zu erleichtern.

5. Lanzette nach Anspruch 2 oder 3, **dadurch gekennzeichnet, dass** die Umhüllung (4) auf einer Umfangsaußenfläche zusätzliche Vorsprünge (13) aufweist, um das Greifen zu erleichtern.

## Revendications

1. Lancette, en particulier pour un dispositif de ponction cutanée d'un patient destiné à prélever un échantillon de sang à des fins diagnostiques, composée
d'un corps (1) en matière plastique où une aiguille (2) est fixée coaxialement,
une pointe (3) de l'aiguille (2) étant recouverte par une gaine (4) moulée d'un seul tenant avec le corps (1) et raccordée de manière sécable à celui-ci,
un élément de guidage (6) coaxial au corps (1) et à la gaine (4),
ledit élément de guidage (6) étant monté de manière à pouvoir coulisser sur un segment de guidage (5) du corps (1) et étant inamoviblement et coaxialement fixé à la gaine (4),
**caractérisée**
**en ce qu'**une surface intérieure de l'élément de guidage (6) et des surfaces extérieures du segment de guidage (5) et de la gaine (4) assurent un guidage par coulissement de l'élément de guidage (6) pendant le montage dudit élément de guidage (6) sur le segment de guidage (5), et en ce qu'une surface intérieure de l'élément de guidage (6) et une surface extérieure du segment de guidage (5) assurent un guidage par coulissement de l'élément de guidage (6) pendant le retrait dudit élément de guidage (6) hors du segment de guidage (5), afin de garantir une mobilité axiale de la gaine (4) par rapport à la pointe (3) pendant le retrait de la gaine (4) hors de la pointe (3).

2. Lancette selon la revendication 1, **caractérisée en ce que** l'élément de guidage forme un capuchon (6) pourvu d'un moyen de maintien sur une surface intérieure, **en ce que** la gaine (4) présente sur sa surface périphérique extérieure des saillies (7) qui s'engagent dans le capuchon (6), et **en ce que** la gaine (4) est pourvue d'un bord de maintien (11) correspondant au moyen de maintien du capuchon (6).

3. Lancette selon la revendication 2, **caractérisée en ce que** le moyen de maintien forme une butée périphérique (10, 14).

4. Lancette selon la revendication 2 ou la revendication 3, **caractérisée en ce que** le capuchon (6) présente sur une surface périphérique extérieure des saillies (12) facilitant la préhension.

5. Lancette selon la revendication 2 ou la revendication 3, **caractérisée en ce que** la gaine (4) présente sur une surface périphérique extérieure des saillies complémentaires (13) facilitant la préhension.
